# EUROPEAN PATENT SPECIFICATION

(11) **EP 3 855 187 B1**
(45) Date of publication and mention of the grant of the patent: **21.05.2025**
(21) Application number: 19861678.1
(22) Date of filing: 09.09.2019
(51) Int. Cl.: G01N 33/96, G01N 33/50, G01N 33/72, A61B 10/00

(54) **ARTIFICIAL FECES, AND METHOD FOR MANAGING ACCURACY OF FECAL OCCULT BLOOD TEST USING SAME**
KÜNSTLICHER STUHL UND VERFAHREN ZUR KONTROLLE DER GENAUIGKEIT EINES TESTS FÜR OKKULTES BLUT UNTER VERWENDUNG DESSELBEN
SELLES ARTIFICIELLES ET PROCÉDÉ DE GESTION DE LA PRÉCISION D'UNE RECHERCHE DE SANG OCCULTE DANS LES SELLES UTILISANT CES DERNIÈRES

(30) Priority: 19.09.2018 JP 2018175109
(43) Date of publication of application: 28.07.2021
(73) Proprietor: EIKEN KAGAKU KABUSHIKI KAISHA, Tokyo 110-8408 (JP)
(72) Inventor: YAMADA, Miyu, Shimotsuga-gun, Tochigi 329-0114 (JP); YASUI, Ryota, Shimotsuga-gun, Tochigi 329-0114 (JP); MIIKE, Akira, Sunto-gun, Shizuoka 411-0951 (JP)
(74) Representative: Simmons & Simmons LLP (Munich)
(86) International application number: PCT/JP2019/035394
(87) International publication number: WO 2020/059563

(56) References cited:
- EP-A1- 0 597 435
- JP-A- 2013 257 216
- JP-A- 2014 043 407
- JP-A- 2014 048 074
- JP-A- H10 319 022
- JP-A- H11 218 533
- JP-A- H11 242 027
- US-B1- 6 933 152
- YASUI RYOTA ET AL: "Novel artificial stool material for external quality assurance (EQA) on a fecal immunochemical test for hemoglobin (FIT): The confirmed utility of stable hemoglobin and an internal standard material", CLINICA CHIMICA ACTA, vol. 483, 16 April 2018 (2018-04-16), AMSTERDAM, NL, pages 76 - 81, XP055916290, ISSN: 0009-8981, Retrieved from the Internet <URL:https://www.sciencedirect.com/science/article/pii/S0009898118301888/pdfft?md5=874bb9523a016538b1019a46b6848159&pid=1-s2.0-S0009898118301888-main.pdf> DOI: 10.1016/j.cca.2018.04.021

## Description

### [Technical Field]

The present invention relates to an artificial stool (artificial feces) in which hemoglobin is stabilized. The present invention relates also to a quality control method for fecal occult blood tests (managing accuracy of fecal occult blood test) using the above artificial stool.

### [Background Art]

The fecal occult blood test has been an important screening test for the medical examination of colon cancer. For fecal occult blood tests, it is desirable to use a certain amount of samples scraped from several portions of the fecal surface. To this end, various types of quantitative stool collection mechanisms including collection instrument and suspension mechanisms including a solution for suspension of feces as well as stool collection devices including a filtration mechanism have been developed. The filtrate of a fecal suspension obtained using such stool collection devices can be applied to an automated analyzer, and therefore the fecal occult blood measurement is generally mass-processed by automation.

The fecal occult blood test, despite its importance, has not yet been standardized. While nationwide surveillance of test values (which may be referred to as a "survey," hereinafter) is being carried out, the current situation is that liquid samples or artificial stools that require preparation for the use at each laboratory are used. The reasons for this may be that the stability of hemoglobin and the like in the stool specimen materials is very poor and it is virtually impossible to distribute and store the stool specimen materials for survey and that the error in the amount of collected stool is large.

For the stool specimen materials for survey among the above, in order to carry out a large-scale survey and obtain highly reliable results, specimen materials that are stable in the component content and capable of mass production are required, but it may not be practical to produce such specimen materials using natural feces. In this context, as substitute for natural feces, ARTIFICIAL stools have been proposed, in which an aqueous solution containing hemoglobin and the like is added and mixed with a base material such as rice flour, wheat flour, potato starch, corn flour, and soy protein (e.g., Patent Documents 1 and 2). Patent Document 3 discloses enzymatically decomposed hemoglobin as a way to stabilize hemoglobin.

### [Prior Art Documents]

### [Patent Documents]

[Patent Document 1] JP10-319022A
[Patent Document 2] JP2003-185654A
[Patent Document 3] JP H11 218533A.

### [Summary of the Invention]

### [Problems to be solved by the Invention]

However, even in the artificial stools of Patent Documents 1 and 2, it cannot be said that the hemoglobin in the artificial stools is sufficiently stabilized, and further improvement of the hemoglobin stability has been desired.

An object of the present invention is therefore to provide an artificial stool in which hemoglobin is stabilized. Another object of the present invention is to provide a quality control method for fecal occult blood tests using the above artificial stool.

### [Means for solving the Problems]

As a result of studies to solve the above problems, the present inventors have found that even though a sugar alcohol or a fragmented product of hemoglobin is not sufficient alone in the effect of stabilizing hemoglobin, these may be used in combination thereby to significantly stabilize hemoglobin, and have accomplished the present invention.

Specifically, the present invention is as described below.
(1) An artificial stool comprising a base material and hemoglobin, the artificial stool further comprising one or more types of sugar alcohols and a fragmented product of hemoglobin.
(2) The artificial stool as described in (1), wherein the sugar alcohol comprises at least one type selected from the group consisting of glycerol, sorbitol, mannitol, erythritol, and xylitol.
(3) The artificial stool as described in (1) or (2), wherein the sugar alcohol comprises a sugar alcohol whose carbon number is four or more.
(4) The artificial stool as described in any one of (1) to (3), wherein the content of the sugar alcohol is 1.5 to 70 mass%.
(5) The artificial stool as described in any one of (1) to (4), wherein the fragmented product of hemoglobin is an enzymatically partial decomposed product of hemoglobin.
(6) The artificial stool as described in any one of (1) to (5), wherein an iron equivalent of the fragmented product of hemoglobin is 2 to 100 µg/g.
(7) The artificial stool as described in any one of (1) to (6), comprising an internal standard material.
(8) The artificial stool as described in (7), wherein the internal standard material is glycerol.
(9) A method of stabilizing hemoglobin in an artificial stool comprising a base material and the hemoglobin, the method comprising including one or more types of sugar alcohols and a fragmented product of hemoglobin in the artificial stool.
(10) A method comprising: collecting the artificial stool as described in any one of (1) to (8); and measuring a collection amount of the hemoglobin in the collected artificial stool.
(11) The method as described in (10), further comprising calculating a content of the hemoglobin in the artificial stool on the basis of the collection amount of the hemoglobin and a collection amount of the artificial stool.
(12) The method as described in (10), wherein the artificial stool is the artificial stool as described in (7) or (8); the method further comprising measuring a collection amount of the internal standard material in the collected artificial stool.
(13) The method as described in (12), further comprising: calculating a collection amount of the artificial stool on a basis of the collection amount of the internal standard material; and calculating a content of the hemoglobin in the artificial stool on the basis of the collection amount of the hemoglobin and the collection amount of the artificial stool.
(14) A quality control method for fecal occult blood tests, the method comprising using the content of the hemoglobin in the artificial stool obtained in the method as described in (11) or (13) as an indicator.

### [Advantageous Effect of the Invention]

The artificial stool of the present invention contains the sugar alcohol and the fragmented product of hemoglobin, and the hemoglobin is therefore significantly stabilized. Moreover, the use of the artificial stool allows the quality control of the fecal occult blood test to be easy.

### [Embodiments for Carrying out the Invention]

Hereinafter, one or more embodiments of the present invention will be described.

### <Artificial stool>

The artificial stool according to an embodiment of the present invention includes a base material and hemoglobin and further includes a sugar alcohol and a fragmented product of hemoglobin.

### 1. Base Material

The type of the base material is not particularly limited, provided that it is a material that allows the physical properties to be approximated to those of feces. Specific examples of the base material include grain flours such as rice flour, wheat flour, barley flour, corn flour, soybean flour, and potato flour; and animal powders such as small fish powder, shellfish powder, and egg yolk powder. These may each be used alone or two or more types may also be used in combination.

The content of the base material in the artificial stool can be appropriately determined based on the physical properties that can be imparted to the artificial stool, and can be, for example, 25 to 70 mass% in an embodiment, 35 to 65 mass% in another embodiment, or 40 to 60 mass% in still another embodiment.

As used in the present specification, the "content" of each component contained in the artificial stool refers to the ratio of the mass of each component to the mass of the artificial stool.

### 2. Hemoglobin

The hemoglobin used in the artificial stool of the present embodiment may be detected in the same manner as that for hemoglobin detected in the fecal occult blood test. More specifically, it may be preferably human hemoglobin.

The content of hemoglobin in the artificial stool may be the same as an amount that is ordinarily detected in feces. For example, the content can be 1 to 400 µg/g in an embodiment or 10 to 200 µg/g in another embodiment.

The content of hemoglobin in the artificial stool can be calculated through collecting the artificial stool, measuring the mass (collection amount) of hemoglobin contained in the artificial stool, and dividing the measured mass of hemoglobin by the mass of the artificial stool. The collection amount of hemoglobin can be measured by the same scheme as that in the fecal occult blood test. More specifically, the collection amount of hemoglobin can be measured by immunological methods such as a latex agglutination reaction method, a gold colloid agglutination reaction method, an immunochromatography method, and an ELISA method; chemical chromogenic reaction methods using the peroxidase-like activity of hemoglobin; or the like.

The marker detected in the artificial stool is hemoglobin, but in addition to hemoglobin, at least one type of other markers may be detected, such as a blood protein such as haptoglobin, hemoglobin-haptoglobin complex, transferrin, α-1 antitrypsin, or α-2 macroglobulin or a protein such as calprotectin.

### 3. Sugar Alcohol

The sugar alcohol in the present embodiment may act synergistically with the fragmented product of hemoglobin, which will be described later, and can stabilize the hemoglobin.

Examples of the sugar alcohol that can be used in the present embodiment include sorbitol, mannitol, xylitol, erythritol, and glycerol. Cyclitol such as inositol or quercitol may also be used.

These sugar alcohols may each be used alone or two or more types may also be used in combination.

From the viewpoint of more effectively achieving the stabilization of hemoglobin synergistically with the fragmented product of hemoglobin, the above sugar alcohol may preferably contain a sugar alcohol whose carbon number is four or more and may further preferably contain a sugar alcohol whose carbon number is five or more.

Aldose and ketose, which are reducing sugars that form an aldehyde group and/or a ketone group in solution, such as glucose and fructose, may react with the hemoglobin in the artificial stool to modify protein and modifies the hemoglobin to form glycated hemoglobin. For example, depending on the type of antibody used in the immunoassay, if the epitope of hemoglobin or its vicinity is modified by a reducing sugar and the structure changes, then the antibody will not be able to react with the hemoglobin contained in the artificial stool, and accurate measurement may not be possible. Accordingly, it is particularly preferred to use a sugar alcohol.

Addition of a sugar alcohol at a high concentration may generate a eutectic product of water and the sugar alcohol, which has a higher eutectic point concentration and a lower eutectic point temperature than those of sugar; therefore, even when stored at a temperature within a range of -10°C to 0°C, ice is less likely to occur and a homogeneous artificial stool may readily be provided.

The important properties required for the artificial stool used for the survey include the homogeneity of the artificial stool and the resistance to freezing. The artificial stool can ordinarily be stored frozen during transportation and storage to ensure the stability of hemoglobin in the artificial stool, but freezing causes the moisture contained in the artificial stool to collect and generate ice, resulting in non-uniformity of the artificial stool, and the hemoglobin may be localized. On the other hand, to ensure the stability of hemoglobin and avoid the non-uniformity of the artificial stool due to freezing, extemporaneous preparation-type artificial stools have been proposed, which are designed such that components such as hemoglobin are freeze-dried, dissolved in water immediately before the use, and mixed with the base material, but errors due to pre-use preparation operations are unavoidable.

In contrast, the artificial stool according to the present embodiment uses a sugar alcohol and therefore has the effects that cohesion and freezing of water are prevented, phase separation that causes localization of hemoglobin is less likely to occur, and the artificial stool can be provided which is still homogeneous even after freezing and thawing. That is, the artificial stool according to the present embodiment can be provided as a ready-to-use artificial stool because the hemoglobin is stabilized and the homogeneity is high even after freezing and thawing, and in this case preparation errors may not occur.

Thus, from the viewpoint of providing an artificial stool having high homogeneity even after freezing and thawing, sorbitol, xylitol, erythritol, and glycerol having high eutectic point concentrations may be preferred, and sorbitol, xylitol, and glycerol may be particularly preferred.

From the above viewpoints, preferred examples of specific sugar alcohols include glycerol, sorbitol, mannitol, erythritol, and xylitol, among which sorbitol, mannitol, erythritol, and xylitol whose carbon number is four or more may be more preferred, sorbitol and xylitol having high eutectic point concentrations may be further preferred, and sorbitol may be particularly preferred because it does not cause the Maillard reaction, does not form glycated hemoglobin, and is inexpensive.

Some sugar alcohols can also be used as internal standard materials, which will be described later (details will be described later).

The lower limit of the content of sugar alcohol in the artificial stool according to the present embodiment may be preferably 1.5 mass%, more preferably 5 mass%, further preferably 10 mass%, and particularly preferably 15 mass% or more. When the content of sugar alcohol is the above lower limit or more, the effect of stabilizing hemoglobin in the artificial stool may be even more significant.

On the other hand, the upper limit of the content of sugar alcohol is not particularly limited, but may be 70 mass% in an embodiment, 50 mass% in another embodiment, or 40 mass% in still another embodiment. Also when the content of sugar alcohol is the above upper limit or less, the effect of stabilizing hemoglobin is sufficiently exhibited according to the present embodiment.

When a sugar alcohol whose carbon number is four or more is contained as the sugar alcohol, the lower limit of the content of sugar alcohol whose carbon number is four or more in the artificial stool may be preferably 1 mass%, further preferably 3 mass%, and particularly preferably 8 mass%. When the content of sugar alcohol whose carbon number is four or more is the above lower limit or more, the effect of stabilizing hemoglobin in the artificial stool may be even more significant.

On the other hand, the upper limit of the content of sugar alcohol whose carbon number is four or more is not particularly limited, but may be 50 mass% in an embodiment, 45 mass% in another embodiment, or 35 mass% in still another embodiment. Also when the content of sugar alcohol whose carbon number is four or more is the above upper limit or less, the effect of stabilizing hemoglobin is sufficiently exhibited according to the present embodiment.

### 4. Fragmented Product of Hemoglobin

The fragmented product of hemoglobin used in the present embodiment may be obtained by fragmenting hemoglobin.

The fragmented product of hemoglobin can act synergistically with the previously described sugar alcohol to stabilize the hemoglobin.

Animals from which the hemoglobin to be fragmented is derived are not limited and may be, for example, humans or non-human vertebrate animals having hemoglobin, such as mammals such as pigs, cows, horses, sheep, goats, and rabbits, birds, or fishes.

Examples of the method of fragmentation include an enzymatic decomposition method and a chemical decomposition method, but the fragmentation may be preferably achieved by the enzymatic decomposition method. The enzyme used in the enzymatic decomposition method is not particularly limited, provided that it is a proteolytic enzyme, and a general-purpose proteolytic enzyme such as pepsin or alcalase can be used.

In the enzymatic decomposition method, it may be preferred to limit the decomposition to partial decomposition from the viewpoint of enhancing the effect of stabilizing hemoglobin. That is, the above fragmented product of hemoglobin may be preferably an enzymatically partial decomposed product of hemoglobin.

The lower limit of the iron equivalent of the fragmented product of hemoglobin in the artificial stool according to the present embodiment may be preferably 2 ug/g, further preferably 10 ug/g, and particularly preferably 20 µg/g. From another aspect, the upper limit of the iron equivalent of the above fragmented product of hemoglobin may be preferably 100 µg/g, further preferably 70 µg/g, and particularly preferably 50 µg/g.

When the iron equivalent of the fragmented product of hemoglobin falls within the above range, the effect of stabilizing hemoglobin by the fragmented product of hemoglobin is even more significant.

Here, in the present embodiment, the iron equivalent of the fragmented product of hemoglobin is the amount of iron ions bound to the fragmented product of hemoglobin via heme.

The iron equivalent of the fragmented product of hemoglobin in the artificial stool can be measured by a known method such as an ortho-phenanthroline colorimetric method or an atomic absorption method. For example, the fraction containing the fragmented product of hemoglobin may be fractionated from the artificial stool by electrophoresis, mass spectrometry, gel filtration chromatography, ultrafiltration, etc. using the molecular weight as an indicator, and the amount of iron in the obtained fraction can be calculated by any of the above methods. A more simple procedure may include measuring the amount of total iron in the artificial stool by any of the above methods, separately measuring/calculating the hemoglobin content, then calculating the amount of iron derived from hemoglobin from the ratio of the amount of iron in hemoglobin (3.39 mg of iron per 1 g of hemoglobin), and subtracting the amount of iron derived from hemoglobin from the amount of total iron to obtain the iron equivalent of the fragmented product of hemoglobin.

The lower limit of the content of the fragmented product of hemoglobin in the artificial stool may be preferably 1 mass part, further preferably 3 mass parts, and particularly preferably 10 mass parts with respect to 1 mass part of hemoglobin. The upper limit of the content of the above fragmented product of hemoglobin may be preferably 80 mass parts, further preferably 60 mass parts, and particularly preferably 40 mass parts with respect to 1 mass part of hemoglobin.

Here, the content of the fragmented product of hemoglobin in the above artificial stool can be calculated through dividing the mass (collection amount) of the fragmented product of hemoglobin in the collected artificial stool by the collection amount of the artificial stool. The collection amount of the fragmented product of hemoglobin can be measured through separating (or fractionating) the fragmented product of hemoglobin and the hemoglobin by electrophoresis, mass spectrometry, gel filtration chromatography, ultrafiltration, etc. using the molecular weight as an indicator and quantifying them. The hemoglobin and the fragmented product of hemoglobin can be separated (or fractionated) from other components using the light absorption of heme as an indicator.

### 5. Internal standard material

The artificial stool according to the present embodiment may preferably contain an internal standard material.

Inclusion of the internal standard material allows the collection amount of the artificial stool to be calculated by measuring the collection amount of the internal standard material when the artificial stool is collected and used for fecal occult blood tests.

In the fecal occult blood test, the reasons why it is difficult to carry out a large-scale survey are that a stool specimen material for survey with excellent reliability (hemoglobin stability) is not provided, that the error in the amount of collected stool is large, etc. The error in the amount of collected stool depends largely on the type of a stool collection device as well as the skill of the stool collection maneuver and it has been very difficult to eliminate the error in the amount of collected stool. In this regard, in a preferred aspect of the present embodiment, even when there is an error in the collection amount of the artificial stool, the value can be corrected because the collection amount of the artificial stool is calculated using the internal standard material.

For the internal standard material that can be used in the present embodiment, it is desired that the collection amount be easy to measure and the internal standard material be stable and do not adversely affect the stability of hemoglobin and the measurement.

More specifically, examples of the internal standard material include glycerol, glycerol-3-phosphate, lactic acid, choline, sugars such as glucose, maltose, and trehalose, 4-aminoantipyrine, p-nitrophenol, p-nitroaniline, and phosphate, which can be easily measured in the field of clinical examination as in the fecal occult blood test. These may each be used alone or two or more types may also be used in combination.

Among these, glycerol, glycerol-3-phosphate, lactic acid, glucose, and 4-aminoantipyrine may be preferred, and glycerol may be particularly preferred because it is excellent in the stability, is easy to measure, is excellent in the sensitivity, can be added at a high concentration, and has an effect of stabilizing hemoglobin.

The collection amount of these internal standard materials can be easily measured by a conventionally known method used in clinical examination facilities.

Sugars including the previously described sugar alcohols can also be used as the internal standard material. Such an internal standard material is easy to measure the collection amount and has an effect of stabilizing hemoglobin, and can be particularly preferably used in the present embodiment. Examples of such sugars that can be used also as the internal standard material include glycerol, glucose, maltose, and trehalose, among which glycerol may be particularly preferred. When glycerol is used as the internal standard material, for example, it is also preferred to use sugar alcohol (such as sorbitol) whose carbon number is four or more together with the glycerol.

The lower limit of the content of the internal standard material in the artificial stool may be preferably 0.5 mass%, more preferably 1 mass%, further preferably 2 mass%, and particularly preferably 4 mass%. When the content of the internal standard material is the above lower limit or more, the measurement of the collection amount of the internal standard material is easier upon collection of the artificial stool, and the collection amount of the artificial stool can be calculated with a high degree of accuracy and precision.

On the other hand, the upper limit of the content of the internal standard material is not particularly limited, and may be 50 mass% in an embodiment, 30 mass% in another embodiment, 20 mass% in still another embodiment, or 10 mass% in yet another embodiment. Also when the content of the internal standard material is the above upper limit or less, the measurement of the collection amount of the internal standard material is easy, the operation of dilution or the like may be omitted upon measurement, and the collection amount of the artificial stool can be easily measured with a high degree of accuracy and precision.

### 6. Other Components

The artificial stool according to the present embodiment can be compounded with water as necessary from the viewpoint of viscosity adjustment and the like. The lower limit of the content of water in the artificial stool can be 0 mass% in an embodiment, 5 mass% in another embodiment, 10 mass% in still another embodiment, or 30 mass% in yet another embodiment. On the other hand, the upper limit of the content of water can be 65 mass% in an embodiment, 60 mass% in another embodiment, or 50 mass% in still another embodiment.

Salts, buffers, etc. can be added to the above water to prepare an aqueous solution such as a salt water solution such as a normal saline solution or a buffer solution containing a buffer. Examples of the salts for appropriate use include dietary salt and phosphate, and examples of the buffer solutions for appropriate use include Good's buffer solution such as HEPES or PIPES, phosphate buffer solution, Tris buffer solution, glycine buffer solution, glycylglycine buffer solution, and a mixed solution of these buffers. The pH of the aqueous solution can be specifically pH 5 to 10 and may be preferably pH 6 to 8 from the viewpoint of the stability of hemoglobin.

Provided that the effects of the present embodiment are not impaired, the artificial stool according to the present embodiment can be appropriately compounded with antiseptics such as sodium azide; colorants; preservatives such as benzoic acid and sorbic acid; and/or fungicides such as orthophenyl phenols, diphenyl, and thiabendazole in addition to the previously described components.

### 7. Method of Producing Artificial stool

The method of producing the artificial stool of the present embodiment is not particularly limited, provided that the above components can be contained, but from the viewpoint of further enhancing the homogeneity in the artificial stool, the method may include dissolving components other than the base material, that is, hemoglobin, a sugar alcohol, and a fragmented product of hemoglobin and, if desired, an internal standard material and other components, in an appropriate solvent such as water, well mixing the obtained solution with the base material, and impregnating the base material with the solution. In this case, the mass ratio of the base material and the solution may be preferably 1:0.5 to 1:1.7, more preferably 1:0.6 to 1:1.6, and particularly preferably 1:0.7 to 1:1.5.

Another method may include mixing the base material and other components and then mixing the obtained mixture and an appropriate solvent such as water.

The homogeneity can be confirmed through appropriately sampling several portions of the obtained artificial stool and measuring the concentration of hemoglobin, internal standard material, etc.

The obtained artificial stool can be stored under a low-temperature condition (e.g., 4°C or lower, preferably -10°C or lower) and can be used after returning the temperature to room temperature or the like for the use.

According to the artificial stool of the embodiments described above, the sugar alcohol and the fragmented product of hemoglobin are contained, and the hemoglobin is therefore significantly stabilized (this corresponds to the method of stabilizing hemoglobin in the artificial stool according to the present invention). Moreover, such an artificial stool is suitably used for the purpose of quality control for fecal occult blood tests.

Furthermore, such an artificial stool can also be provided as a ready-to-use artificial stool and does not require the extemporaneous preparation in which the user dissolves components in water and mixes them with the base material immediately before the use.

The content of hemoglobin contained in the artificial stool, during each of the packaging, transportation, storage at each laboratory, and use, may ideally have to be the same as the hemoglobin content at shipping. In the stability test, however, the test has been conducted under a very severe test condition in order to clarify the time constraint and the differences between systems. It has been confirmed that assuming a slightly severe condition in which the test is conducted at room temperature (about 20°C to 25°C) during the above packaging, transportation, storage at each laboratory, use, etc., the content of hemoglobin is stable for a certain period of time at the room temperature (data are omitted).

### <Quality control method for Fecal Occult Blood Tests and Method of Evaluating Stool collection device>

The quality control method for fecal occult blood tests and the method of evaluating the stool collection device according to the present embodiment uses the artificial stool according to the above embodiments.

In the present embodiment, the hemoglobin in the artificial stool is highly stabilized, and the quality control for the fecal occult blood tests is therefore achieved by using such an artificial stool as a specimen material.

Here, in the present specification, the collection amount of the artificial stool (also referred to as a stool collection amount) refers to the mass of the artificial stool collected in the quality control method according to the present embodiment.

Also in the present specification, the collection amount of each component (such as hemoglobin or an internal standard material) contained in the artificial stool refers to the mass of each component contained in the above collected artificial stool. In the present embodiment, the collection amount of hemoglobin or an internal standard material in the artificial stool can be obtained, for example, through suspending the collected artificial stool in a solution that contains a buffer solution or the like, measuring the concentration of the hemoglobin or the internal standard material in the suspension by a known method, and using the obtained concentration and the amount of solution.

According to the present embodiment, it is also possible to identify the cause of an error from the data of the collection amount of hemoglobin and the collection amount of the artificial stool, and the quality control is thus easier. That is, when using the hemoglobin collection amount, the total testing error including the error in the stool collection stage and the measurement error in the hemoglobin becomes clear. On the other hand, when using the hemoglobin content calculated through dividing the hemoglobin collection amount by the corresponding collection amount of each artificial stool, the stool collection error is corrected, and if there is an error, it becomes clear that the error is purely due to the reagent and measurement device for measuring the hemoglobin. It is thus possible to understand at which stage of the test a problem occurs, and the countermeasures become concrete.

A preferred aspect of the quality control method according to the present embodiment can be performed, for example, as follows (Quality control method (1)).
(1a) The artificial stool according to the above embodiments is collected, and the collection amount of hemoglobin in the collected artificial stool is measured.
(1b) The content of hemoglobin in the artificial stool is calculated based on the collection amount of hemoglobin obtained in the above (1a) and the collection amount of the artificial stool.
(1c) In the quality control method for fecal occult blood tests, the content of hemoglobin in the artificial stool obtained in the above (1b) is used as an indicator.

Here, for the collection amount of the artificial stool in the above (1b), for example, the mass of the collected artificial stool can be measured, and the obtained measurement value can be used as the collection amount of the artificial stool in the above (1b).

When the stool collection amount can be regarded as a constant value owing to the mechanism of the stool collection device or the like used for collecting the artificial stool, such a value may be used as the collection amount of the artificial stool in the above (1b).

When using an artificial stool that contains an internal standard material, the collection amount of the internal standard material in the collected artificial stool may be measured, and the collection amount of the artificial stool may be calculated based on the measured collection amount of the internal standard material (Quality control method (2), which will be described later).

In the above (1a) to (1c), the same operator (e.g., a laboratory) may perform all the steps collectively. Examples of such a quality control method include management of variations between devices and daily variations in the laboratory.

In the above (1a) to (1c), two or more operators may also share any of the steps. For example, a laboratory of fecal occult blood tests may perform only the above (1a) or the above (1a) and (1b), and an External quality control provider may analyze the obtained results and perform the above (1b) and (1c) (or the above (1c)). Examples of such method include quality control between different stool collection devices and a control survey.

Another preferred aspect of the quality control method according to the present embodiment can be performed, for example, as follows (Quality control method (2)).
(2a) The artificial stool (which further contains an internal standard material) according to the above embodiments is collected, and the collection amount of hemoglobin and the collection amount of the internal standard material in the collected artificial stool are measured.
(2b) The collection amount of the artificial stool is calculated based on the collection amount of the internal standard material obtained in the above (2a).
(2c) The content of hemoglobin in the artificial stool is calculated based on the collection amount of hemoglobin obtained in the above (2a) and the collection amount of the artificial stool obtained in the above (2b).
(2d) In the quality control method for fecal occult blood tests, the content of hemoglobin in the artificial stool obtained in the above (2c) is used as an indicator.

In (2a) to (2d) of the quality control method (2), as in the previously described quality control method (1), the same operator may perform all the steps collectively, or two or more operators may also share any of the steps.

When two or more operators share any of the steps, for example, a laboratory of fecal occult blood tests may perform only the above (2a) or the above (2a) and (2b) or (2a) to (2c), and an quality control provider may analyze the obtained results and perform the above (2b) to (2d) (or the above (2c) and (2d) or only the above (2d)). Additionally or alternatively, a operator other than the laboratory or quality control provider may carry out the above (2b) and/or (2c).

When using the artificial stool containing an internal standard material for the quality control, the collection amount of the artificial stool may be used as an indicator.

For example, a reference example of a quality control method can be performed as follows (Quality control method (3)).
(3a) The artificial stool (which further contains an internal standard material) according to the above embodiments is collected, and the collection amount of the internal standard material in the collected artificial stool is measured.
(3b) The collection amount of the artificial stool is calculated based on the collection amount of the internal standard material obtained in the above (3a).
(3c) In the quality control method for fecal occult blood tests, the collection amount of the artificial stool obtained in the above (3b) is used as an indicator.

In (3a) to (3c) of the quality control method (3), as in the previously described quality control methods (1) and (2), the same operator may perform all the steps collectively, or two or more operators may also share any of the steps.

When two or more operators share any of the steps, for example, a laboratory of fecal occult blood tests may perform only the above (3a) or the above (3a) and (3b), and a quality control provider may analyze the obtained results and perform the above (3b) and (3c) (or only the above (3c)).

In the quality control method for fecal occult blood tests, a modified example when using the collection amount of the artificial stool as an indicator may include performing steps corresponding to (2a) and (2b) of the previously described quality control method (2) to calculate the collection amount of the artificial stool and then using the obtained collection amount of the artificial stool as an indicator for the quality control method for fecal occult blood tests.

The method using the collection amount of the artificial stool as an indicator is not limited to the quality control of the fecal occult blood test and may also be used, for example, for the evaluation of the stool collection device. More specifically, when designing or experimentally producing a stool collection device or testing the performance of a completed stool collection device, the performance or the like of the stool collection device can be evaluated through collecting a stool using the above artificial stool, obtaining the collection amount of the artificial stool by measurement or calculation, and using the collection amount of the artificial stool as an indicator. In particular, it may be preferred to evaluate the performance or the like of a stool collection device through collecting a stool using the above artificial stool (which includes an internal standard material), measuring the collection amount of the internal standard material thereby to calculate an amount of the artificial stool collected in the stool collection device, and using the obtained amount of the artificial stool as an indicator. A preferred example of such a method may be, for example, an aspect of calculating the collection amount of the artificial stool through the same steps as those in the above quality control method (2) or the above quality control method (3) and then using the obtained collection amount of the artificial stool as an indicator for the method of evaluating the stool collection device.

The evaluation of a stool collection device can include controlling the accuracy of the stool collection device.

According to the quality control methods for fecal occult blood tests as described above, it becomes easy to assure/control the quality of the fecal occult blood test because the hemoglobin in the artificial stool is highly stabilized. Furthermore, when the artificial stool contains an internal standard material, the quality control becomes easier and the stool collection device can be evaluated.

It should be appreciated that the embodiments heretofore explained are described to facilitate understanding of the present invention

### [Examples]

Hereinafter, the present invention will be described in more detail by presenting production examples, testing examples, etc.

### <Production Examples> Preparation of Artificial stools

Rice flour (available from Ioki New Torii Co., Ltd.) was washed with deionized water, filtered, and dried. The obtained washed rice flour was used as the base material.

On the other hand, a 50 mM HEPES buffer solution was made to contain the following components to prepare an additive solution.
- Human hemoglobin A0 (available from Sigma-Aldrich Co. LLC): 100 µg/mL (in the additive solution)
- Sugar alcohol (sugars): Concentrations in the artificial stools are listed in the tables.
- Fragmented product of hemoglobin derived from pigs obtained using a proteolytic enzyme (available from ILS Inc., Hemeron 2HiWS, iron content of 2.1 mass%): Concentrations in the artificial stools are listed in the tables.
- Sodium azide (available from FUJIFILM Wako Pure Chemical Corporation): 0.09 mass% (in the artificial stools)

Here, the fragmented product of hemoglobin is one with which a broad band can be confirmed at a position of a molecular weight of 3 kDa to 9 kDa by SDS-PAGE analysis, and is distinguished from unfragmented human hemoglobin (both the α chain and the β chain are about 16 kDa).

The above base material (washed rice flour) and the additive solution were mixed at a mass ratio of 1:1.5 to prepare a paste-like artificial stool. The obtained artificial stool was stored at -20°C until it was used for an accelerated test.

In the accelerated test, after thawing the artificial stool, samples were each collected before and after the accelerated test (45°C: 1 day, 3 days, and 7 days) and subjected to the hemoglobin measurement as described below.

### <Testing Example 1> Measurement of Hemoglobin in Artificial stools

After collecting samples of about 10 to 20 mg from several portions of each artificial stool before or after the accelerated test and measuring the collection amount (mg), the samples were put into a test tube containing 4 mL of a buffer solution and stirred to suspend the samples in the buffer solution. The suspension in the test tube was centrifuged and the supernatant was used for the hemoglobin measurement.

OC-Hemodia Auto III 'Eiken' (available from EIKEN CHEMICAL CO., LTD.) was used as the hemoglobin measurement reagent, and the measurement was performed using OC sensor DIANA (available from EIKEN CHEMICAL CO., LTD.).

On the basis of the obtained hemoglobin measurement value (ng/mL), the value was divided by the collection amount (mg) of the artificial stool, which had been preliminarily measured, and corrected with the buffer solution amount (4 mL) to calculate a corrected hemoglobin concentration (µg/g stool) in accordance with the following equation. Corrected hemoglobin concentration = (Measured value/Collection amount) × Buffer solution amount

From the obtained result, the stability of hemoglobin was evaluated as the residual hemoglobin ratio through dividing the corrected hemoglobin concentration after the accelerated test by the concentration before the accelerated test.

The results are listed in Tables 1 to 5. For the samples listed in the same table, the accelerated tests were conducted in parallel.

**[Table 1]**

| Sample No. | Composition | | | | Residual hemoglobin ratio | | |
|---|---|---|---|---|---|---|---|
| | Glycerol (mass% ) | Sorbitol (mass% ) | Fragmented product of Hb | | 45°C 1 day | 45°C 3 days | 45°C 7 days |
| | | | Iron equivalen t (µg/g stool) | To Hb (mass parts) | | | |
| Sample 1-1 | - | - | - | - | 11.6% | 0.4% | 0.9% |
| Sample 1-2 | 5.2 | | | | 17.0% | 0.3% | 1.0% |
| Sample 1-3 | 10.4 | | | | 16.8% | 0.4% | 0.9% |
| Sample 1-4 | 15.6 | | | | 19.1% | 1.3% | 1.3% |
| Sample 1-5 | - | | 3.3 | 3 | 73.6% | 5.4% | 1.5% |
| Sample 1-6 | 5.2 | | | | 76.0% | 16.9% | 2.5% |
| Sample 1-7 | 10.4 | | | | 77.4% | 32.9% | 3.1% |
| Sample 1-8 | 15.6 | | | | 84.7% | 38.7% | 5.1% |
| Sample 1-9 | - | 10.4 | - | - | 38.9% | 4.7% | 1.7% |
| Sample 1-10 | 5.2 | | | | 38.8% | 8.7% | 1.5% |
| Sample 1-11 | 10.4 | | | | 42.8% | 5.7% | 2.0% |
| Sample 1-12 | 15.6 | | | | 44.5% | 10.9% | 2.3% |
| Sample 1-13 | - | | 3.3 | 3 | 88.0% | 58.8% | 25.5% |
| Sample 1-14 | 5.2 | | | | 92.0% | 63.2% | 33.4% |
| Sample 1-15 | 10.4 | | | | 91.2% | 66.7% | 30.0% |
| Sample 1-16 | 15.6 | | | | 90.8% | 65.1% | 31.4% |

As listed in Table 1, by using glycerol and the fragmented product of hemoglobin together, the hemoglobin was stabilized depending on the concentration of glycerol. Moreover, by using glycerol and sorbitol together as sugar alcohols, the stability of hemoglobin became more significant.

**[Table 2]**

| Sample No. | Composition | | | | Residual hemoglobin ratio |
|---|---|---|---|---|---|
| | Glycerol (mass% ) | Sorbitol (mass% ) | Fragmented product of Hb | | 45°C 7 days |
| | | | Iron equivalent (µg/g stool) | To Hb (mass parts) | |
| Sample 2-1 | - | - | - | - | 0.0% |
| Sample 2-2 | 5.2 | - | - | - | 0.0% |
| Sample 2-3 | 5.2 | 2.6 | | | 0.2% |
| Sample 2-4 | 5.2 | 5.2 | | | 0.2% |
| Sample 2-5 | 5.2 | 10.4 | | | 0.7% |
| Sample 2-6 | 5.2 | 26 | | | 32.3% |
| Sample 2-7 | 5.2 | - | 3.3 | 3 | 2.2% |
| Sample 2-8 | 5.2 | 2.6 | | | 7.6% |
| Sample 2-9 | 5.2 | 5.2 | | | 27.6% |
| Sample 2-10 | 5.2 | 10.4 | | | 51.4% |
| Sample 2-11 | 5.2 | 26 | | | 84.6% |

As listed in Table 2, also for sorbitol, an effect of stabilizing the hemoglobin depending on the concentration was recognized.

**[Table 3]**

| Sample No. | Composition | | | | Residual hemoglobin ratio |
|---|---|---|---|---|---|
| | Glycerol (mass% ) | Sorbitol (mass% ) | Fragmented product of Hb | | 45°C 7 days |
| | | | Iron equivalent (µg/g stool) | To Hb (mass parts) | |
| Sample 3-1 | - | - | - | - | 0.0% |
| Sample 3-2 | 5.2 | - | - | - | 0.0% |
| **Sample** 3-3 | | | 3.3 | 3 | 2.2% |
| Sample 3-4 | | | 11 | 10 | 19.2% |
| Sample 3-5 | | | 22 | 20 | 23.5% |
| Sample 3-6 | | | 44 | 40 | 11.7% |
| Sample 3-7 | | | 88 | 80 | 4.5% |
| Sample 3-8 | | 10.4 | - | - | 0.7% |
| Sample 3-9 | | | 3.3 | 3 | 51.4% |
| Sample 3-10 | | | 11 | 10 | 64.7% |
| Sample 3-11 | | | 22 | 20 | 68.4% |
| Sample 3-12 | | | 44 | 40 | 65.4% |
| Sample 3-13 | | | 88 | 80 | 35.8% |

As listed in Table 3, the fragmented product of hemoglobin and the sugar alcohol were used together thereby to stabilize the hemoglobin, and when the iron equivalent of the fragmented product of hemoglobin was 2 to 70 µg/g, the effect of stabilizing hemoglobin was more significant. Moreover, when the content of the fragmented product of hemoglobin was 1 to 60 mass parts with respect to 1 mass part of hemoglobin, the effect of stabilizing hemoglobin was more significant.

**[Table 4]**

| Sample No. | Composition | | | | | Residual hemoglobin ratio | |
|---|---|---|---|---|---|---|---|
| | Glycerol (mass %) | Sugars | | Fragmented product of Hb | | 45°C 1 day | 45°C 3 days |
| | | | (mas s%) | Iron equivalent (µg/g stool) | To Hb (mass parts) | | |
| Sample 4-1 | - | - | - | - | - | 1.3% | 1.3% |
| Sample 4-2 | 2.6 | - | - | - | - | 0.6% | 1.3% |
| Sample 4-3 | | Sorbitol | 10.4 | | | 7.8% | 3.7% |
| Sample 4-4 | | Xylitol | 10.4 | | | 5.2% | 1.8% |
| Sample 4-5 | | Fructose | 10.4 | | | 5.5% | 1.2% |
| Sample 4-6 | | - | - | 3.3 | 3 | 30.8% | 16.3% |
| Sample 4-7 | | Sorbitol | 10.4 | | | 80.1% | 77.7% |
| Sample 4-8 | | Xylitol | 10.4 | | | 78.3% | 72.1% |
| Sample 4-9 | | Fructose | 10.4 | | | 37.0% | 7.8% |

As listed in Table 4, also when xylitol was used as the sugar alcohol other than sorbitol, the use together with the fragmented product of hemoglobin exhibited a significant effect of stabilizing the hemoglobin.

**[Table 5]**

| Sample No. | Composition | | | | | Residual hemoglobin ratio |
|---|---|---|---|---|---|---|
| | Glycerol (mass% ) | Sugars | | Fragmented product of Hb | | 45°C 7 days |
| | | | (mass %) | Iron equivalent (µg/g stool) | To Hb (mass parts) | |
| Sample 5-1 | - | - | - | - | - | 1.8% |
| Sample 5-2 | 5.2 | - | - | | | 1.6% |
| Sample 5-3 | | Sorbit ol | 15.6 | | | 6.5% |
| Sample 5-4 | | Xylitol | 15.6 | | | 4.8% |
| Sample 5-5 | | Erythri tol | 15.6 | | | 4.7% |
| Sample 5-6 | | Glycer ol | 15.6 | | | 2.9% |
| Sample 5-7 | - | - | - | 22 | 20 | 11.2% |
| Sample 5-8 | 5.2 | - | - | | | 16.4% |
| Sample 5-9 | | Sorbit ol | 15.6 | | | 69.9% |
| Sample 5-10 | | Xylitol | 15.6 | | | 54.6% |
| Sample 5-11 | | Erythri tol | 15.6 | | | 65.6% |
| Sample 5-12 | | Glycer ol | 15.6 | | | 32.7% |

As listed in Table 5, also when xylitol, erythritol, or glycerol was used as the sugar alcohol, an excellent effect of stabilizing the hemoglobin was recognized through the use together with the fragmented product of hemoglobin.

### <Testing Example 2> Evaluation of Uniformity of Artificial stools

For each of the artificial stools of Sample 5-8 to Sample 5-12 to which glycerol was added among the samples listed in Table 5 and the artificial stool of Sample 5-13 for which fructose was used as substitute for the sugar alcohol, after taking samples of about 10 to 20 mg from three portions of the artificial stool immediately after the preparation and measuring the collection amount (mg), the samples were put into a test tube containing 4 mL of a buffer solution and stirred to disperse the samples in the buffer solution. The test tube was subjected to centrifugation and the supernatant was used for glycerol measurement.

In addition, for each of the artificial stools of the same Sample 5-8 to Sample 5-13, freezing at -20°C and thawing at 25°C were repeated five times without performing an accelerated test, samples of about 10 to 20 mg were taken from ten portions of the artificial stool after such freezing and thawing, and a supernatant for glycerol measurement was obtained in the same manner as above.

A glycerol measurement reagent was prepared by preliminarily mixing Reagent 1 and Reagent 2 of EKDIA XL 'Eiken' TG II (available from EIKEN CHEMICAL CO., LTD.) at a volume ratio of 3:1, and automated clinical chemistry analyzers 7170s and 7180 (both available from Hitachi High-Tech Corporation) were used for the measurement.

The obtained measurement value was converted to a glycerol concentration, and this concentration was divided by the collection amount of the artificial stool, which had been preliminarily measured, and corrected with the buffer solution amount to calculate a corrected glycerol concentration (mg/g stool). For a series of three samples or a series of ten samples obtained from the same artificial stool, an average value and a standard deviation of the corrected glycerol concentration were calculated, from which a coefficient of variance (CV, %) was calculated.

The results are listed in Table 6. If the corrected glycerol concentration is approximately constant, it is considered to be uniform. (See JP2014-43407A).

**[Table 6]**

| Sam ple No. | Composition | | | | | When prepared (N=3 Sampling) | | | After repeating freezing and thawing 5 times (N=10 Sampling) | | |
|---|---|---|---|---|---|---|---|---|---|---|---|
| | Glycer ol (mas s%) | Sugars | | Frag produc mented t of Hb | | Amou nt of Gly in artifici al stool (mg/ 9 stool ) | Sta nda rd dev iati on | CV( %) | Amou nt of Gly in artifici al stool (mg /g stool ) | Sta nda rd dev iati on | CV( %) |
| | | | (m ass %) | Iron equival ent (µ 9/9 stool) | To Hb (mas s parts ) | | | | | | |
| Sam ple 5-8 | 5.2 | - | - | 22 | 20 | 52.34 | 0.1 5 | 1.2 % | 49.61 | 0.8 4 | 7.0 % |
| Sam ple 5-9 | | Sor bito l | 15. 6 | | | 54.19 | 0.1 9 | 1.5 % | 54.01 | 0.2 4 | 1.8 % |
| Sam ple 5-10 | | Xyli tol | 15. 6 | | | 54.01 | 0.0 7 | 0.6 % | 54.36 | 0.2 1 | 1.6 % |
| Sam ple 5-11 | | Eryt hrit ol | 15. 6 | | | 55.05 | 0.2 0 | 1.5 % | 54.23 | 0.3 2 | 2.4 % |
| Sam ple 5-12 | | Gly cer ol | 15. 6 | | | 208.0 5 | 0.3 7 | 0.7 % | 210.6 7 | 1.1 6 | 2.3 % |
| Sam ple 5-13 | | Fru ctos e | 15. 6 | | | 52.97 | 0.0 8 | 0.6 % | 57.63 | 1.6 3 | 12. 5% |

As listed in Table 6, the uniformity of the artificial stools using a sugar alcohol was high even after the freezing and thawing were repeated five times.

### [Industrial Applicability]

According to the present invention, an artificial stool in which hemoglobin is highly stabilized can be provided, and such an artificial stool is very useful for the quality control of the fecal occult blood test including a control survey.

## Claims

1. An artificial stool comprising a base material and hemoglobin, the artificial stool further comprising one or more types of sugar alcohols and a fragmented product of hemoglobin.

2. The artificial stool according to claim 1, wherein the sugar alcohol comprises at least one type selected from the group consisting of glycerol, sorbitol, mannitol, erythritol, and xylitol.

3. The artificial stool according to claim 1 or 2, wherein the sugar alcohol comprises a sugar alcohol whose carbon number is four or more.

4. The artificial stool according to any one of claims 1 to 3, wherein a content of the sugar alcohol is 1.5 to 70 mass%.

5. The artificial stool according to any one of claims 1 to 4, wherein the fragmented product of hemoglobin is an enzymatically partial decomposed product of hemoglobin.

6. The artificial stool according to any one of claims 1 to 5, wherein an iron equivalent of the fragmented product of hemoglobin is 2 to 100 µg/g.

7. The artificial stool according to any one of claims 1 to 6, comprising an internal standard material.

8. The artificial stool according to claim 7, wherein the internal standard material is glycerol.

9. A method of stabilizing hemoglobin in an artificial stool comprising a base material and the hemoglobin, the method comprising
including one or more types of sugar alcohols and a fragmented product of hemoglobin in the artificial stool.

10. A method comprising:
collecting the artificial stool according to any one of claims 1 to 8; and
measuring the collection amount of the hemoglobin in the collected artificial stool.

11. The method according to claim 10, further comprising:
calculating a content of the hemoglobin in the artificial stool on a basis of the collection amount of the hemoglobin and a collection amount of the artificial stool.

12. The method according to claim 10, wherein
the artificial stool is the artificial stool according to claim 7 or 8,
the method further comprising:
measuring a collection amount of the internal standard material in the collected artificial stool.

13. The method according to claim 12, further comprising:
calculating a collection amount of the artificial stool on a basis of the collection amount of the internal standard material; and
calculating a content of the hemoglobin in the artificial stool on a basis of the collection amount of the hemoglobin and the collection amount of the artificial stool.

14. A quality control method for fecal occult blood tests, the method comprising obtaining the content of the hemoglobin in the artificial stool by the method according to claim 11 or 13, and using said content as an indicator.

## Patentansprüche

1. Künstlicher Stuhl, der ein Basismaterial und Hämoglobin umfasst, wobei der künstliche Stuhl ferner eine oder mehrere Arten von Zuckeralkoholen und ein fragmentiertes Produkt von Hämoglobin umfasst.

2. Künstlicher Stuhl nach Anspruch 1, wobei der Zuckeralkohol mindestens eine Art umfasst, die aus der Gruppe bestehend aus Glycerin, Sorbit, Mannit, Erythrit, und Xylit ausgewählt ist.

3. Künstlicher Stuhl nach Anspruch 1 oder 2, wobei der Zuckeralkohol einen Zuckeralkohol umfasst, dessen Kohlenstoffzahl vier oder mehr beträgt.

4. Künstlicher Stuhl nach einem der Ansprüche 1 bis 3, wobei der Gehalt an Zuckeralkohol 1,5 bis 70 Masse-% beträgt.

5. Künstlicher Stuhl nach einem der Ansprüche 1 bis 4, wobei das fragmentierte Produkt von Hämoglobin ein enzymatisch teilweise abgebautes Produkt von Hämoglobin ist.

6. Künstlicher Stuhl nach einem der Ansprüche 1 bis 5, wobei ein Eisengehalt des fragmentierten Produkts von Hämoglobin 2 bis 100 µg/g beträgt.

7. Künstlicher Stuhl nach einem der Ansprüche 1 bis 6, umfassend ein internes Standardmaterial.

8. Künstlicher Stuhl nach Anspruch 7, wobei das interne Standardmaterial Glycerin ist.

9. Verfahren zur Stabilisierung von Hämoglobin in einem künstlichen Stuhl, der ein Basismaterial und das Hämoglobin umfasst, wobei das Verfahren umfasst: das Einbeziehen einer oder mehrerer Arten von Zuckeralkoholen und eines fragmentierten Produkts von Hämoglobin in den künstlichen Stuhl.

10. Verfahren, umfassend: das Sammeln des künstlichen Stuhls nach einem der Ansprüche 1 bis 8; und das Messen der Sammelmenge des Hämoglobins im gesammelten künstlichen Stuhl.

11. Verfahren nach Anspruch 10, ferner umfassend: das Berechnen eines Gehalts des Hämoglobins im künstlichen Stuhl auf Basis der Sammelmenge des Hämoglobins und einer Sammelmenge des künstlichen Stuhls.

12. Verfahren nach Anspruch 10, wobei der künstliche Stuhl der künstliche Stuhl nach Anspruch 7 oder 8 ist, das Verfahren ferner umfassend: das Messen einer Sammelmenge des internen Standardmaterials im gesammelten künstlichen Stuhl.

13. Verfahren nach Anspruch 12, ferner umfassend: das Berechnen einer Sammelmenge des künstlichen Stuhls auf Basis der Sammelmenge des internen Standardmaterials; und das Berechnen eines Gehalts des Hämoglobins im künstlichen Stuhl auf Basis der Sammelmenge des Hämoglobins und der Sammelmenge des künstlichen Stuhls.

14. Qualitätskontrollverfahren für Tests auf okkultes Blut im Stuhl, wobei das Verfahren das Erhalten des Gehalts des Hämoglobins im künstlichen Stuhl durch das Verfahren nach Anspruch 11 oder 13 umfasst und diesen Gehalt als Indikator verwendet.

## Revendications

1. Selles artificielles comprenant un matériau de base et de l'hémoglobine, les selles artificielles comprenant en outre un ou plusieurs types d'alcools de sucre et un produit fragmenté de l'hémoglobine.

2. Selles artificielles selon la revendication 1, dans lesquelles l'alcool de sucre comprend au moins un type choisi dans le groupe constitué par le glycérol, le sorbitol, le mannitol, l'érythritol et le xylitol.

3. Selles artificielles selon la revendication 1 ou 2, dans lesquelles l'alcool de sucre comprend un alcool de sucre dont le nombre de carbones est égal ou supérieur à quatre.

4. Selles artificielles selon l'une des revendications 1 à 3, dans lesquelles la teneur en alcool de sucre est comprise entre 1,5 et 70 % en masse.

5. Selles artificielles selon l'une des revendications 1 à 4, dans lesquelles le produit fragmenté de l'hémoglobine est un produit d'hémoglobine partiellement décomposé par voie enzymatique.

6. Selles artificielles selon l'une des revendications 1 à 5, dans lesquelles l'équivalent en fer du produit fragmenté de l'hémoglobine est compris entre 2 et 100 µg/g.

7. Selles artificielles selon l'une des revendications 1 à 6, comprenant un matériau standard interne.

8. Selles artificielles selon la revendication 7, dans lesquelles le matériau standard interne est le glycérol.

9. Méthode de stabilisation de l'hémoglobine dans des selles artificielles comprenant un matériau de base et l'hémoglobine, comprenant
inclure un ou plusieurs types d'alcools de sucre et un produit fragmenté d'hémoglobine dans les selles artificielles.

10. Procédé comprenant :
recueillir les selles artificielles selon l'une des revendications 1 à 8 ; et
mesurer la quantité d'hémoglobine dans les selles artificielles recueillies.

11. Procédé selon la revendication 10, comprenant en
calculer la teneur en hémoglobine des selles artificielles sur la base de la quantité d'hémoglobine recueillie et de la quantité de selles artificielles recueillie.

12. Procédé selon la revendication 10, dans lequel
les selles artificielles sont les selles artificielles selon la revendication 7 ou 8,
le procédé comprenant en outre
mesurer une quantité de prélèvement de l'étalon interne dans les selles artificielles recueillies.

13. Procédé selon la revendication 12, comprenant en outre :
calculer une quantité de selles artificielles sur la base de la quantité de selles du matériau standard interne ; et
calculer la teneur en hémoglobine des selles artificielles sur la base de la quantité d'hémoglobine recueillie et de la quantité de selles artificielles recueillie.

14. Procédé de contrôle de qualité pour les tests de sang occulte dans les selles, la méthode comprenant l'obtention de la teneur en hémoglobine dans les selles artificielles par la méthode selon la revendication 11 ou 13, et l'utilisation de cette teneur comme indicateur.
